# EUROPÄISCHE PATENTSCHRIFT

(11) **EP 3 341 173 B1**
(45) Veröffentlichungstag und Bekanntmachung des Hinweises auf die Patenterteilung: **02.10.2019**
(21) Anmeldenummer: 16760015.4
(22) Anmeldetag: 24.08.2016
(51) Int. Cl.: B29B 7/46, B29B 7/90, B29C 71/00, A61K 6/00, A61K 6/027, B29C 48/00, B29C 48/30, C08G 77/00, B29B 7/72, B29B 7/84, B29B 9/06, B29C 35/08, B29L 31/00, B29K 101/10, B29C 48/40, B29C 48/53, B29C 48/76, B29C 48/92

(54) **MISCH- UND FORMVERFAHREN FÜR GEFÜLLTE DUROMERE AUS ORGANISCH VERNETZBAREN KOMPOSITMASSEN, INSBESONDERE FÜR DENTALE ZWECKE**
MIXING AND MOLDING METHOD FOR FILLED THERMOSETS FROM ORGANICALLY CROSSLINKABLE COMPOSITE MATERIALS, IN PARTICULAR FOR DENTAL PURPOSES
PROCÉDÉ DE MÉLANGE ET DE MISE EN FORME POUR DUROMÈRES CHARGÉS EN MASSES COMPOSITES ORGANIQUEMENT RÉTICULABLES, EN PARTICULIER À DES FINS DENTAIRES

(30) Priorität: 28.08.2015 DE 102015114397
(43) Veröffentlichungstag der Anmeldung: 04.07.2018
(73) Patentinhaber: Wolter, Herbert, 97941 Tauberbischofsheim (DE)
(72) Erfinder: WOLTER, Herbert, 97941 Tauberbischofsheim (DE); HOFFMANN, Jeanette, 93051 Regensburg (DE); HÄUSLER, Florian, 33619 Bielefeld (DE)
(74) Vertreter: Strehl Schübel-Hopf & Partner
(86) Internationale Anmeldenummer: PCT/EP2016/070001
(87) Internationale Veröffentlichungsnummer: WO 2017/036885

(56) Entgegenhaltungen:
- EP-A2- 0 150 674
- DE-A1-102005 018 351
- JP-A- 2011 020 307
- US-A- 5 232 960
- US-A- 5 877 232
- US-A1- 2006 108 706
- US-A1- 2010 060 134
- US-B1- 6 388 001

## Beschreibung

Die vorliegende Erfindung betrifft die Herstellung von geformten Kompositen aus mit Füllstoffen befüllten, organisch vernetzbaren Reaktivharzen sowie deren Aushärtung zu Duromeren.

Für prothetische Versorgungen von defekten oder verloren gegangenen Zähnen werden in den meisten Fällen Kronen, Brücken, Inlays, Onlays, Veneers etc. eingesetzt. Der Trend geht seit einigen Jahren immer stärker in die Richtung der digitalen und automatisierten Fertigung solcher Versorgungen. Die Fertigung erfolgt von der digitalen Abdrucknahme der Gebisssituation des Patienten über die virtuelle Modellation der Versorgung mittels entsprechender CAD/CAM-Software bis hin zum Beschleifen/Fräsen eines entsprechenden Materialblocks. Dabei kommen je nach Anwendung unterschiedliche Materialien, wie z.B. Keramiken, Metalle, Kunststoffe und Wachse zum Einsatz. Die CAD/CAM-Blöcke können "einfarbig", d.h. monochromatisch oder "mehrfarbig" bzw. mit Transluzenzverlauf, d.h. polychromatisch gestaltet sein. Aktuell liegt der Fokus vieler Dentalfirmen auf der Entwicklung unterschiedlichster Kompositmaterialien für eine Anwendung im CAD/CAM-Bereich. Einige Firmen haben schon Materialien auf dem Markt, wobei ein großer Teil dieser Materialien nur für provisorische Zwecke verwendet wird.

Damit die Massen noch in Formen vergossen werden können, muss die Viskosität der Materialien für die Herstellung von Komposit-basierten CAD/CAM-Blöcken gering sein. Eine hohe Festigkeit und Abrasionsresistenz geht aber häufig mit einem hohen Füllstoffgehalt der Komposite einher. Solche Komposite müssen in mehreren Arbeitsschritten hergestellt werden, die sehr zeit- und damit kostenintensiv sind: Um tatsächlich eine homogene Mischung der Ausgangsmaterialien (Reaktivharz und Füllstoff) zu erhalten, werden je nach Füllstofftyp, Füllstoffgehalt und Partikelgröße unterschiedliche Mischverfahren wie z.B. Dreiwalzwerk (kontinuierlich), Planetenmischer oder Speedmixer (beide nicht-kontinuierlich) eingesetzt und ggf. miteinander kombiniert. Aufgrund der notwendigen Kombination der Mischverfahren handelt es sich um ein nicht-kontinuierliches Gesamtverfahren, was einen hohen zeitlichen Aufwand bei der Herstellung bedeutet.

Die so hergestellten Komposite besitzen eine so hohe Viskosität, dass sie nur durch Kneten, also in aufwändiger Handarbeit, ggf. unter Pressen, weiter in die notwendige Form gebracht werden können. Dabei ist es ist so gut wie unmöglich, blasenfreie Formkörper herzustellen. Auch das Ausstreichen oder Gießen größerer Platten ist kein geeignetes Verfahren, da entstehende Blasen nicht aus der gegossenen oder ausgestrichenen Masse entfernt werden können. Derartige Platten müssen nach dem Härten noch in die finalen Formteile zerteilt werden. Dabei gehen große Anteile an Material verloren. Aufgrund ihrer großen Zähigkeit können solche Komposite auch nicht vermittels sonstiger automatisierbarer Verfahren wie Stranggusspressen oder dergleichen in die gewünschte Form überführt werden.

Für die Herstellung Komposit-basierter CAD/CAM-Blöcke ist der Fachmann daher einem Dilemma ausgesetzt: Wählt er als Herstellungsverfahren das Vergießen der Komposite in Blockformen, weisen diese aufgrund des reduzierten Füllstoffgehaltes häufig nicht die gewünschten mechanischen Eigenschaften auf. Besitzen die Komposite dagegen den gewünschten, hohen Füllstoffgehalt, so müssen die Blöcke aufwändig in Handarbeit, unter Umständen mit Pressen, hergestellt oder in die Form großer Platten gebracht werden, wobei letztere nach dem Härten durch eine Abfolge mehrdimensionaler Schnitte in die entsprechenden Blockformen gesägt/zerteilt werden müssen. Neben der bereits oben erwähnten Problematik, dass die bei diesen Verfahren erhältlichen Formkörper kaum blasenfrei hergestellt werden können, ist das letztgenannte außerdem durch die aufwändigen Sägeschritte sowie den besonderen Kostenaufwand für teure, schnell verschleißende Sägeblätter gekennzeichnet; außerdem ist der Materialverlust hoch. Bei hoher Viskosität ist auch nur schwer eine gute Formtreue zu erreichen, weil z.B. scharfe Kanten schlecht formbar sind.

Dieses Dilemma existiert nicht nur im Dentalbereich, sondern betrifft die Herstellung von zu Duromeren aushärtenden, hochgefüllten Kunststoffen (Reaktivharzen im Sinne der untenstehenden Definition) ganz allgemein.

Angesichts dieser Situation haben sich die Erfinder die Aufgabe gestellt, ein kostengünstiges, automatisierbares Verfahren aufzufinden, mit dem sich Komposite aus mit Füllstoff gefüllten Reaktivharzmatrices blasenfrei erzeugen und in die gewünschte Form bringen lassen, wobei der Füllstoffgehalt der Komposite so hoch gewählt werden kann, dass das ausgehärtete Produkt gute mechanische Eigenschaften aufweist, die insbesondere für die Anwendung im Dentalbereich benötigt werden.

Geeignete, insbesondere für die genannten dentalen Zwecke nutzbare Kompositmaterialien, auch als Kompositmassen bezeichnet, sind in der Regel eine Kombination aus einem Reaktivharz, das als Matrix fungiert, und einer Füllung aus einem oder mehreren Füllstoffen, gegebenenfalls unter Zusatz weiterer Additive. Die Reaktivharzmatrix besteht aus einem organischen oder anorganisch-organischen Material, das durch Licht und/oder Wärme und/oder redoxinduziert einer Vernetzung vorhandener organischer Gruppen unterworfen werden kann und dabei aushärtet. Bei der Aushärtung der Kompositmassen erhält man in der Regel Duromere, d.h. Materialien, die sich nicht durch Erwärmen wieder erweichen oder aufschmelzen lassen.

Als Reaktivharze für die Durchführung der vorliegenden Erfindung eignen sich grundsätzlich organisch polymerisierbare Systeme, z.B. organische Monomere/Oligomere oder anorganisch-organische Hybridmaterialien mit organischen Gruppen, die sich unter der Einwirkung von Licht und/oder Wärme und/oder auf chemischem Wege organisch polymerisieren lassen und dabei in Duromere übergehen. Bei der Polymerisationsreaktion handelt es sich in der Regel um eine Polyreaktion, bei der reaktionsfähige Doppelbindungen oder Ringe unter dem Einfluss von Wärme, Licht, ionisierender Strahlung oder chemisch (über eine Redoxreaktion) in Polymere übergeht (englisch "additon polymerization" oder "chain growth polymerization"). Die organische Polymerisation erfolgt vorzugsweise über nichtaromatische C=C-Doppelbindungen, die stärker bevorzugt aktiviert sind, und dabei insbesondere über (Meth-)Acryl- und/oder Norbornenylgruppen, oder über Gruppen mit gespannten Ringsystemen, insbesondere Epoxidgruppen.

Unabhängig davon, ob die Reaktivharze aus rein organischem oder einem anorganisch-organischen Hybridmaterial bestehen, können enthaltene Ringstrukturen wie Epoxidgruppen im Rahmen einer (z.B. Epoxid-)Polymerisation zum alleinigen Aufbau einer organischen Polymerstruktur und damit zur Härtung genutzt werden. Gleiches gilt für Gruppen, die nichtaromatische C=C-Doppelbindungen enthalten. In einer Variante können aber auch polymerisierbare Ausgangsmaterialien eingesetzt werden, die sowohl gespannte Ringstrukturen wie Epoxidgruppen als auch nichtaromatische C=C-Doppelbindungen enthalten, oder sie können unterschiedlich reaktive Ringe und/oder C=C-Doppelbindungen enthalten. Bei Einsatz entsprechender Initiatoren können auf diesem Wege eine thermisch initiierte und eine photochemisch initiierte Polymerisationsreaktion zeitlich gesteuert nacheinander ablaufen, so dass man die Härtung in zwei Schritte, eine (moderate) Vorhärtung und eine Endhärtung (endgültige Aushärtung) unterteilen kann, wobei die Vorhärtung bewirkt, dass ein standfestes Material entsteht, das sich transportieren und ggf. zwischenlagern lässt.

Ringstrukturen wie Epoxidgruppen können unter üblichen, allgemein bekannten Bedingungen kationisch ringöffnend polymerisiert werden (thermisch bzw. photoinitiiert nach Zugabe geeigneter Initiatoren wie Lewis- bzw. Brönsted-Säuren bzw. Verbindungen die solche Säuren freisetzen). Weiterhin können die Ringstrukturen durch di-, tri- bzw. tetrafunktionelle Verbindungen wie z. B. Di-, Tri- bzw. Tetraamine, Di-, Tri- bzw. Tetrathiole, Di-, Tri- bzw. Tetraanhydride, Di-, Tri- bzw. Tetracarbonsäuren vernetzt und somit ausgehärtet werden. Bei den nichtaromatischen C=C-Doppelbindungen handelt es sich um Gruppierungen, die unter dem Einfluss von Wärme, Licht, ionisierender Strahlung oder redoxinduziert (z.B. mit einem Initiator (Peroxid oder dgl.) und einem Aktivator (Amin oder dgl.)) einer organischen Vernetzung unterworfen werden und dabei in Polymere übergehen können.

Beispiele für doppelbindungshaltige Gruppen sind solche, die eine (oder mehrere) einer Michaeladdition zugängliche Doppelbindungen aufweisen wie Styryle, Norbornenyle oder (Meth)Acrylsäure-Derivate; es kann sich aber auch um Vinyl- oder Allylgruppen handeln. Unter (Meth-)Acrylderivaten bzw. (Meth-)Acrylsäurederivaten sind dabei vor allem die (Meth-)Acrylate, (Meth-)Acrylamide und deren Thio-Analoga zu verstehen. Werden anorganisch-organische Hybridmaterialien eingesetzt, macht eine solche Polymerisationsreaktion eine z.B. lichtinduzierte nachträgliche Vernetzung des Kieselsäurepolykondensats möglich.

Beispiele für duromer härtbare organische Matrices sind monomere oder oligomere (Meth)acrylate, die zu Duromeren aushärten können. Soll ein anorganisch-organisches Hybridmaterial eingesetzt werden, kann dieses beispielsweise aus einem oder mehreren organisch modifizierten Kieselsäure(hetero)polykondensat(en) bestehen oder diese(s) enthalten, wie sie beispielweise insbesondere in der DE 4011044, der EP 0450624, der DE 4133494, der DE 4310733, der DE 4416857.8, der DE 19627198, der DE 19910895, der DE 10349766.8 und EP1914260, der DE 102005018351, der DE 102005018059, der DE 102011054440.2, der DE 102011053865.8, der DE 102012109685.6, der DE 102013108594.6 und der DE 102014115751.6 beschrieben sind. Dabei werden die notwendigen chemischen/ physikalischen Eigenschaften dieser Materialien je nach Anforderungsprofil gewählt.

Selbsthärtende anorganisch-organische Hybridpolymere, die über einen Redoxmechanismus härten, sind beispielsweise in EP 0618242 beschrieben.

Es gibt zusätzliche und alternative Wege, um organische Vernetzungen zu ermöglichen. Eine spezielle Form der Härtung nutzt nicht nur die Polymerisationsreaktion (Polyaddition) der C=C-Doppelbindungen als solcher wie oben erläutert. Möglich ist nämlich auch eine Umsetzung der diese Doppelbindungen enthaltenden Verbindungen mit Di- oder höheren Aminen oder Di- oder höheren Thiolen über eine Michael-Addition (Thiol-En-Umsetzung bzw. die analoge Umsetzung mit Aminen). Möglich ist dies mit Di-, Tri, Tetra- oder sogar noch höher funktionalisierten Aminen oder Mercaptanen, wobei die Reaktion mit Aminen (nur) dann gelingt, wenn die C=C-Doppelbindungen in aktivierter Form vorliegen, beispielsweise als Acryl- oder Methacrylgruppen (darunter (Meth)acrylatgruppen).

Die genannte Härtung (Polyaddition) kann gegebenenfalls vollständig an die Stelle der Polymerisationsreaktion der C=C-Doppelbindungen (der "chain growth polymerization") treten; es bildet sich dabei ein etwas lockereres organisches Netzwerk, weil S-(Kohlenwasserstoff)-S-Brücken bzw. N-(Kohlenwasserstoff)-N-Brücken ausgebildet werden. Sie kann aber auch zusätzlich erfolgen, indem die Menge an Di- bzw. Polythiolen oder Di- bzw. Polyaminen so niedrig gewählt wird, dass C=C-Doppelbindungen im Harz verbleiben, die anschließend auf üblichem Wege nachgehärtet werden können.

Damit eine spätere Härtung möglich ist, sollte das Harz in vielen Fällen ein Initiatorsystem zur lichtinduzierten, thermisch induzierten und/oder redoxinduzierten Polymerisation der darin enthaltenen organisch polymerisierbaren Gruppen aufweisen. Es kann auch weitere Zusätze enthalten, beispielsweise einen Katalysator für eine zuvor abgelaufene hydrolytische Kondensation im Falle eines Kieselsäure(hetero)polykondensates als Harzmaterial.

US 5 232 960 A offenbart ein Verfahren zum Mischen und Formen von Kompositen aus einer Reaktivharzmatrix, die beim Aushärten duromere Eigenschaften annimmt, und einem Füllstoff. Den Erfindern ist es gelungen, ein Misch- und Formverfahren für gefüllte Duromere aus organisch vernetzbaren Kompositmassen wie oben definiert bereitzustellen. Das Verfahren lässt sich sowohl auf die Erzeugung noch unvernetzter Massen anwenden, die beispielsweise anschließend für eine spätere Formgebung und Aushärtung abgefüllt werden, als auch auf die Erzeugung von endausgehärteten Formkörpern. Es handelt sich dabei um ein vorzugsweise kontinuierlich arbeitendes Extrusionsverfahren, das zumindest die folgenden Schritte aufweist:
(a) Dosieren eines flüssigen oder pastösen Reaktivharzes in einen Extruder,
(b) Zugabe von Füllstoff zu dem im Extruder befindlichen Reaktivharz,
(c) Vermischen der Komponenten unter Ausbildung eines Komposits,
(d) Fördern des Komposits in einen Bereich des Extruders, in dem es aufgestaut wird,
(e) Entgasen des aufgestauten Komposits,
(f) Fördern des entgasten Komposits durch eine geeignet geformte Düse aus dem Extruder heraus,
(g) Härten des aus dem Extruder getretenen Komposits durch Belichten und/oder Erwärmen, und
(h) Zerteilen des Strangs in die gewünschte Form,
wobei die Schritte (g) und (h) in beliebiger Reihenfolge durchgeführt werden können.

Extrusionsverfahren sind aus der Kunststofftechnik bekannt. Vor allem werden sie in der Kautschukindustrie und zur Herstellung von PVC und Polyethylen/Polypropylen genutzt. Alle thermoplastischen Materialien können in Pulver- oder Granulatform in einen Extruder gefüllt, aufgeschmolzen und gegebenenfalls mit Zusätzen vermischt extrudiert werden; Bedingung ist hierfür nur eine hohe Viskosität im Schmelzzustand. Diese ist notwendig, damit die aus dem Werkzeug austretende Schmelze ihre gegebene Gestalt für kurze Zeit beibehält und nicht zerfließt, bevor sie durch Abkühlung wieder erstarrt.

Die vorliegende Erfindung ist im Gegensatz hierzu geeignet, duromere Komposite zu erzeugen.

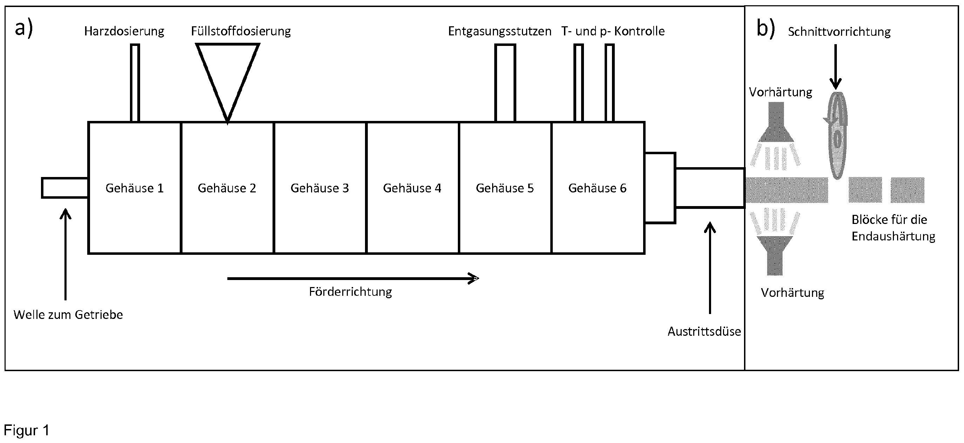

Das erfindungsgemäße Verfahren erfolgt größtenteils in einem Extruder, in dem alle verfahrenstechnischen Prozesse ablaufen. Dieser ist schematisch in **Teil a) der** **Figur 1** dargestellt, wobei die dort gezeigte Aufteilung in Gehäuse und deren Anzahl optional ist (der Extruder kann auch nur ein Gehäuse aufweisen oder aus einer anderen Zahl von Modulen zusammengesetzt sein, die jeweils mit einem Gehäuse versehen sind). Der Extruder kann im Ganzen oder teilweise beheizt (z.B. elektrisch beheizt) oder - z.B. mit Wasser - gekühlt werden, um so eine optimale Prozesstemperatur einzustellen. Dabei ist es empfehlenswert, dass der Extruder außenseitig auf einer Temperatur gehalten wird, die mindestens etwa 10K, vorzugsweise mindestens 30K unter der Temperatur liegt, bei der das Reaktivharz ggf. wärmebedingt zu polymerisieren beginnt.

Der Extrusionsprozess bewirkt dem Grunde nach die - in der Regel kontinuierliche - Förderung eines Stranges aus noch unvernetztem, homogen vermischtem Komposit. Das austretende Material kann aber natürlich auch unmittelbar hinter der Düse abgefangen und in andere Formen gebracht werden. Außerdem kann das Komposit erfindungsgemäß bei Bedarf sofort und räumlich unmittelbar hinter der Austrittsdüse oder aber entweder zu einem anderen Zeitpunkt und/oder an einem anderen Ort vor- und/oder endverfestigt werden.

Nachstehend sollen die einsetzbaren Ausgangsmaterialien sowie die einzelnen Verfahrensschritte näher erläutert werden.

Als duromer härtendes Reaktivharz wird ein solches eingesetzt, wie es oben definiert ist. Die Viskosität des (ungefüllten) Reaktivharzes kann von flüssig bis pastös gewählt werden. Viskositäten bis zu 500 oder bis zu 1000 Pas (bei 25°C) sind möglich und können bei geeigneter Verfahrensführung sogar noch deutlich überschritten werden. Ist ein Harz zu hochviskos für eine gewählte Vorrichtung, kann eine Temperaturerhöhung beim Einfüllen hilfreich sein. In der Regel wird jedoch mit deutlich niedrigeren Viskositäten gearbeitet, z.B. 1 bis 200 Pas.

Das duromer härtende Reaktivharz kann ungeteilt oder unterteilt in zwei oder mehrere Komponenten in den Extruder eingebracht werden. Besteht das Reaktivharz beispielsweise aus einer epoxidhaltigen und/oder C=C-doppelbindungshaltigen Komponente sowie einem Thiol oder einer (oder mehreren) sonstigen Verbindung(en), die an das Epoxid oder die C=C-Doppelbindung addieren kann, können die epoxid- und/oder C=C-doppelbindungshaltige Komponente und die additionsfähige Verbindung gemeinsam oder getrennt - im letzteren Fall über zwei Einfüll-Einrichtungen - zudosiert werden. Wird ein über eine Redoxreaktion härtendes Harz eingesetzt, können ggf. der Aktivator (z.B. ein aromatisches Amin) und der Initiator (z.B. ein Peroxid), jeweils in einen Teil des Reaktivharzes eingemischt, ebenfalls über zwei getrennte Einfüll-Einrichtungen zudosiert werden, was aber nicht zwingend ist. In einer spezifischen Alternative hierzu kann zur Verhinderung einer vorzeitig schon im Extruder ablaufenden Härtung des Komposits der Großteil des Harzes mit nur einem der beiden notwendigen Additive, dem Aktivator oder dem Initiator, gemischt eindosiert und sodann mit Füllstoff vermischt werden, während eine kleine Menge an flüssigem oder pastösem Harz, mit dem noch fehlenden Additiv vermischt, dem dabei entstandenen Komposit über eine weitere Dosierungseinrichtung erst kurz vor dem Austritt aus der Düse zugesetzt wird.

In einer spezifischen Ausgestaltung der Erfindung können Zusätze, die nicht nur, aber insbesondere für die Härtungsreaktion des Reaktivharzes benötigt oder gewünscht werden, in verkapselter Form zugegeben werden. Es ist aus dem Stand der Technik bekannt, mit Flüssigkeit gefüllte Kapseln zu erzeugen, siehe z.B. DE 10 2009 019 370 A1. Feste Zusätze können nach der in DE 10 2012 202 069 A1 für die Verkapselung von Schwefel beschriebenen Methode verkapselt werden. In der erstgenannten Druckschrift handelt es sich bei dem Verkapselungsmaterial in der Regel um ein organisch modifiziertes Kieselsäure(hetero)-polykondensat, das vorzugsweise zusätzlich über organische Gruppen vernetzt ist. Das Verkapselungsmaterial der DE 10 2012 202 069 A1 ist ein Kieselsäurepolykondensat, das durch organische Gruppen modifiziert sein kann, aber nicht muss. Geeignete Kapseln sind aber nicht auf Kieselsäure(hetero)polykondensate als Kapselmaterial beschränkt; gefüllte Kapseln lassen sich alternativ beispielsweise auch aus oder mit rein organisch polymerisierbaren Materialien wie (Meth-)Acrylaten herstellen. Derartige Kapseln sind unter den Scherkräften, die im erfindungsgemäß einzusetzenden Extruder auftreten, in der Regel nicht stabil bzw. können vom Fachmann ohne weiteres mit einer Kapselwand-Dicke hergestellt werden, die unter diesen Scherkräften nicht stabil ist. Aus den im Extruder zerbrechenden/zerquetschten Kapseln kann der Inhalt austreten; er wird im Extruder homogen mit den anderen Komponenten vermischt, so dass es trotz des Kapsel-Einsatzes Fällen zu einer homogen ablaufenden Polymerisationsreaktion kommt.

Als Zusätze, die in verkapselter Form zugegeben werden können, eignen sich beispielsweise Initiatoren, Katalysatoren und/oder Aktivatoren, soweit sie für die jeweilige Polymerisationsreaktion benötigt werden oder aus anderen Gründen eingesetzt werden sollen.

Beispielsweise können bei einer redox-induzierten Härtung der Initiator und/oder der Aktivator in Kapseln zugesetzt werden. Verkapselter Initiator und verkapselter Aktivator können in ein und dasselbe Material oder jeweils in unterschiedliche Teile des Materials eingearbeitet werden, die, wie oben beschrieben, über zwei getrennte Einfüll-Einrichtungen zudosiert werden. Statt dessen können sowohl der verkapselte Initiator als auch der verkapselte Aktivator in nur ein einziges Material eingearbeitet werden. Auch in diesen Fällen läuft die Härtung des Komposits keinesfalls vorzeitig ab, da Initiator und Aktivator erst innerhalb des Extruders in Kontakt miteinander kommen.

Verkapselte Zusätze sind aber keinesfalls auf redox-induzierte Härtungen beschränkt. Sie können in allen Ausgestaltungen der Erfindung eingesetzt werden, in denen ein solcher Zusatz für die jeweilige Polymerisationreaktion, ggf. aber auch für andere Zwecke, benötigt wird oder gewünscht ist. So kann beispielsweise der Initiator, der für eine lichtinduzierte und/oder wärmeinduzierte Epoxidhärtung eingesetzt werden soll, in verkapselter Form in das Komposit eingearbeitet werden. Wird nicht nur die Polymerisationsreaktion (Polyaddition) von C=C-Doppelbindungen, sondern auch eine Umsetzung der diese Doppelbindungen enthaltenden Verbindungen mit Di- oder höheren Aminen oder Di- oder höheren Thiolen über eine Michael-Addition (Thiol-En-Umsetzung bzw. die analoge Umsetzung mit Aminen) genutzt, wie oben erläutert, kann die zusätzliche Amin- oder Thiol-Komponente ebenfalls in verkapselter Form zugesetzt werden.

Das Reaktivharz kann gegebenenfalls auch bereits einen Teil des Füllstoffs enthalten, sofern das dabei erhaltene Komposit noch eine verfahrensgeeignete Viskosität wie oben definiert aufweist.

Der Füllstoff kann aus einem einzigen Material bestehen; alternativ kann er aus mehreren Füllstoffkomponenten mit unterschiedlicher Größe und/oder Zusammensetzung und/oder Form in variablen Anteilen bestehen. Die Füllstoffe können insbesondere partikelförmig und/oder faserförmig (insbesondere Kurzfasern) sein. Als Füllstoffe eignen sich z.B. diejenigen, die in DE 196 43 781, DE 198 32 965, DE 100 184 05, DE 100 41 038, DE 10 2005 061 965 sowie DE 10 2005 018 305 beschrieben sind. In der Regel handelt es sich bei dem oder den Füllstoffen um anorganische Materialien.

Gut geeignet sind nanopartikuläre Füllstoffe oder eine Kombination solcher Füllstoffe verschiedener Größe oder verschiedener Zusammensetzung, ggf. in Kombination mit weiteren, bekannten Füllstoffen wie partikulären Dentalgläsern, z.B. Ba-Sr-Aluminiumborosilikaten. Unter "nanopartikulär" ist dabei zu verstehen, dass die Füllstoffe einen Durchmesser oder ihren größten Durchmesser im Bereich von unter 1000 nm aufweisen.

Sehr gut geeignet sind SiO₂Partikel, die man beispielsweise nach bekannten Sol-Gel-Verfahren erhalten kann und die dann eine sehr enge Durchmesserverteilung aufweisen können. Diese oder auch anders zusammengesetzte Nanopartikel können auf ihrer Oberfläche modifiziert, z.B. silanisiert sein, um ihre Oberflächeneigenschaften an diejenigen der Matrix anzupassen.

Die nanopartikulären Füllstoffe können allein oder in Kombination mit anderen Füllstoffen für die Zwecke der vorliegenden Erfindung eingesetzt werden. Verwendbar als weitere Füllstoffe sind z.B. Makrofüller (z.B. aus Glas, Keramik oder Quartz, Teilchengrößen zwischen 2 bis 50 µm), homogene Mikrofüller (beispielsweise aus pyrogener Kieselsäure, Teilchengrößen ca. 0,02 bis 0,06, vorzugsweise ca. 0,04 µm), inhomogene Mikrofüller (Beispiel: ein Teil der pyrogenen Kieselsäure liegt als Splitterpolymerisat vor), Hybridfüller (Mischungen von Makro- und Mikrofüllern) oder Feinsthybridfüller (z.B. Mischungen aus Aerosil und Ba- oder Sr-Glas mit Teilchengrößen im Bereich von etwa 1 bis 5 µm). Sehr gut geeignet für die vorliegende Erfindung sind beispielsweise Dentalgläser mit Teilchendurchmessern von ca. 0,4 bis 20 µm, bevorzugt von ca. 1-5 µm.

Das Verhältnis der Füllstoffe untereinander kann beliebig gewählt werden. Günstig sind Gewichtsanteile des nanopartikulären Füllstoffs von etwa 5 bis etwa 60 Gew.-%, bezogen auf das Gesamtgewicht an Füllstoff im Komposit. Besonders günstig sind Anteile von über 5 bis 30 Gew.-%. In solchen Ausgestaltungen können die nanopartikulären Füllstoffe in den Hohlräumen oder Lücken zwischen engen, möglicherweise sogar annähernd dichtesten (Kugel-)Packungen von größeren Füllstoffteilchen liegen, insbesondere, wenn die größeren Füllstoffteilen eine annähernd sphärische Gestalt besitzen. Es hat sich herausgestellt, dass beim Einsatz von Anteilen in diesem Bereich besonders hoch gefüllte Komposite erhältlich sind, die eine besonders geringe Schrumpfung und eine besonders hohe Abrasionsfestigkeit aufweisen.

Es ist möglich, dem Reaktivharz zusätzlich zu dem (meist anorganischen) Füllstoff wie oben beschrieben ein festes Harz mit thermoplastischen Eigenschaften wie z.B. PMMA (Polymethylmethacrylat) (letzteres z.B. in Pulverform) zuzusetzen, das noch reaktive, licht- und/oder wärmehärtende Gruppen wie oben definiert (z.B. Methacrylgruppen) tragen kann, aber nicht muss. Dieses Harz ist bei der Temperatur, bei der es in den Extruder gefüllt wird, fest (z.B. pulver- oder granulatförmig); im Extruder wird es nicht plastifiziert. Das entstehende Komposit wird beim Austritt aus dem Extruder durch Licht und/oder Wärme verfestigt oder gehärtet, behält aber (wohl aufgrund der enthaltenen thermoplastischen Partikel) in manchen Fällen bis zu einem gewissen Grad thermoplastische Eigenschaften. Hier wird als Reaktivharz vorzugsweise ein flüssiges oder pastöses, rein organisches, polymerisierbares Monomer oder Oligomer oder eine Mischung mehrerer solcher Monomere und/oder Oligomere eingesetzt. Das Verfahren dieser Alternative umfasst die folgenden Schritte:
(a) Dosieren eines oder mehrerer flüssiger oder pastöser organischer, polymerisierbarer Monomere und/oder Oligomere in einen Extruder,
(b) Zugabe eines festen thermoplastischen Materials sowie mindestens eines Füllstoffs zu dem im Extruder befindlichen Reaktivharz,
(c) Vermischen der Komponenten mit Hilfe von einer oder mehreren Extruderschnecken unter Ausbildung eines Komposits bei einer Temperatur, bei der das thermoplastische Material fest bleibt,
(d) Fördern des Komposits in einen Bereich des Extruders, in der es aufgestaut wird,
(e) Entgasen des aufgestauten Komposits,
(f) Fördern des entgasten Komposits durch eine geeignet geformte Düse aus dem Extruder heraus,
(g) Verfestigen oder Härten des aus dem Extruder getretenen Komposits durch Belichten und/oder Erwärmen, und
(h) Zerteilen des Strangs in die gewünschte Form,
wobei die Schritte (g) und (h) in beliebiger Reihenfolge durchgeführt werden können.

Das thermoplastische Material wird in diesem speziellen Verfahren anders als in "klassischen" Extruder-Verfahren für thermoplastische Materialien zwar mit Hilfe des Extruders innig mit den anderen Komponenten - dem Reaktivharz in Form eines flüssigen oder pastösen organischen, polymerisierbaren Monomers und/oder Oligomers und dem Füllstoff - vermischt, aber nicht zum Erweichen oder Schmelzen gebracht. Im Extruder wird vielmehr ein durch Licht und/oder Wärme verfestigbares bzw. härtbares Komposit gebildet, das sodann nach dem Austritt aus dem Extruder gehärtet bzw. verfestigt wird.

In dieser spezifischen Ausgestaltung kann ein Reaktivharz gewählt werden, das als solches duromer oder thermoplastisch aushärten würde. Ein duroplastisch aushärtenden Reaktivharz ist bevorzugt.

Je nach dem vorgesehenen speziellen Verwendungszweck können dem Komposit außerdem geeignete Additive wie Initiatoren, Färbemittel (Farbstoffe oder Pigmente), Oxidationsinhibitoren, Polymerisationsinhibitoren (für die Vermeidung einer vorzeitigen Polymerisation), Verlaufsmittel, UV-Absorber, Stabilisatoren, mikrobiozide Wirkstoffe oder dergleichen zugesetzt werden, wie es dem Fachmann bekannt ist. Beispiele für Polymerisationsinitiatoren sind Initiatoren für die radikalische Polymerisation, und zwar für die thermische Härtung wie Peroxide (z.B. Dibenzoylperoxid) oder Photoinitiatoren wie Benzophenon, Campherchinon oder Kombinationen von α-Diketonen mit Aminen als Reduktionsmittel, wie beispielsweise aus der DE 199 03 177 C2 bekannt. Für die duale Aushärtung von radikalisch und kationisch polymerisierbaren Systemen können insbesondere Diaryliodonium- oder Triarylsulfoniumsalze zugesetzt werden, für die die vorgenannte Druckschrift ebenfalls Beispiele angibt.

Alternativ zu teilchenförmigem Füllstoff ist die Verwendung von Fasern möglich. Diese richten sich bei der Passage durch den Extruder in etwa parallel zur Schneckenachse aus, wodurch das entstehende Produkt anisotrope Festigkeitseigenschaften, und dabei insbesondere eine hohe Festigkeit in Längsrichtung, erhalten kann.

Das Mischungsverhältnis von Harz zu Füllstoff wird je nach Bedarf gewählt; wie oben erwähnt, sollte der Füllstoffgehalt im Falle der Herstellung von Formteilen für die CAD-CAM-Bearbeitung im Dentalbereich hoch sein. In der vorliegenden Erfindung weisen die Komposite Füllstoffgehalte im Bereich von 45 bis 90 Gew.-%, und vorzugsweise zwischen 60 und 72 Gew.-% auf.

In Schritt (a) des erfindungsgemäßen Verfahrens wird das Reaktivharz in den Extruder eingebracht. Vor allem dann, wenn das Harz thermisch induziert polymerisieren und aushärten kann, muss vermieden werden, dass es sich während des erfindungsgemäßen Verfahren bis auf die Temperatur erwärmt, bei der die Polymerisationsreaktion einsetzt, um Verklumpungen zu vermeiden. Die Dosierung des Harzes in den Extruder erfolgt daher insbesondere bei thermisch härtbaren Harzen, aber auch im Übrigen vorzugsweise druckfrei, besonders bevorzugt mit Hilfe einer Dosierpumpe, in der das Material keiner Scherung unterworfen wird. Dies gelingt beispielsweise drucklos durch die Verwendung einer (z.B. volumetrisch arbeitenden) Schlauchpumpe. Die Verwendung einer Schlauchpumpe hat den zusätzlichen Vorteil, dass ein direkter Kontakt von bewegten Pumpenteilen mit dem Harz vermieden wird. Sofern das Harz scherunempfindlich ist, beispielsweise weil es ausschließlich durch Licht und/oder redoxinduziert aushärtet, und/oder sofern die verwendete Pumpe ausreichend gekühlt wird, kann stattdessen aber auch eine andere Pumpe, beispielsweise eine Zahnradpumpe, eingesetzt werden.

Die vorstehenden Erläuterungen gelten auch für die Alternative, bei der in Schritt (a) flüssiges oder pastöses organisches Monomer/Oligomer eindosiert wird.

Füllstoffe können sehr unterschiedliche Eigenschaften besitzen. Dentale Füllstoffe wie Dentalgläser besitzen häufig ein schlechtes Fließverhalten, was die Dosierung erschwert. Die Zugabe des Füllstoffs gemäß Schritt (b) kann insbesondere bei problematisch zu handhabenden Füllstoffen beispielsweise gravimetrisch über einen Einlauftrichter und gegebenenfalls ein Rührwerk erfolgen. Gleiches gilt für die Zugabe des thermoplastischen Polymer-Pulvers und des Füllstoffs gemäß der oben beschriebenen Alternative. Dabei werden das thermoplastische Pulver und der Füllstoff vorzugsweise vorgemischt und dann gemeinsam zudosiert. Es ist in einer weniger bevorzugten Alternative aber auch möglich, das thermoplastische Material und den Füllstoff über eine getrennte Zudosierungs-Einrichtung in den Extruder zu füllen.

In den nachfolgenden Abschnitten des Extruders werden die beiden Komponenten Harz und Füllstoff homogen vermischt (Schritt (c)) und Richtung Austrittsdüse gefördert (Schritt (d)). Das Vermischen erfolgt während des Fördervorgangs; hierfür kann die Extruderschnecke bei Bedarf spezielle Mischelemente aufweisen, was jedoch im Allgemeinen nicht erforderlich ist. Außerdem kann das Material gegebenenfalls zusätzlich geknetet werden. Hierfür enthält die Extruderschnecke optional geeignet ausgebildete Knetelemente.

Um das Komposit zu entgasen (Schritt e), wird es vor der Düse mit Hilfe einer spezifischen Schneckenkonfiguration aufgestaut. Der Druckaufbau kann beispielsweise über eine Verengung des Gangvolumens der Schnecke / eine Querschnittverengung erzeugt werden. Über eine Bohrung im Gehäuse in diesem Bereich kann mittels Vakuumpumpe ein Unterdruck angelegt werden, um Blasen/Lufteinschlüsse zu minimieren. Dabei ist darauf zu achten, dass Komposite mit sehr hohen Füllstoffgehalten empfindlich gegenüber einem zu starken Unterdruck sind und zum Zerfallen neigen. Dies liegt möglicherweise daran, dass niedrigermolekulare Komponenten des Harzes bei sehr niedrigen Drücken abgezogen werden und die verbleibende Harzmasse nicht ausreicht, den Füllstoff zu binden. Zumindest bei sehr hohen Füllstoffgehalten (z.B. über 70 Gew.-%, zumindest bei über 75 Gew.-%) ist es daher empfehlenswert, den Druck um nicht mehr als 800 mbar, vorzugsweise nicht mehr als 650 mbar (hPa) unter den Normaldruck abzusenken.

Das verdichtete und entgaste Komposit wird sodann durch eine geeignete Düse extrudiert (Schritt f). Die Düse hat die Maße, die dem Querschnitt des austretenden Strangs entsprechen. Will man beispielsweise CAD/CAM-Blöcke für die Herstellung von Zahnersatz erzeugen, empfiehlt sich ein eckiger Querschnitt mit z.B. 12 x 14 mm² Kantenlänge. Selbstverständlich sind andere Düsengeometrien möglich, beispielsweise rund. Optional ist es möglich, dass die Düse einen variablen Querschnitt besitzt, der in geeigneter Weise, beispielsweise elektronisch gesteuert, verändert werden kann. Mit einer solchen Ausgestaltung ist es z.B. möglich, Stränge zu produzieren, die an späteren Schnitt- oder Bruchstellen stellen bereits bei der Extrusion einen kleineren Querschnitt aufweisen, ohne dass diese Stellen ausgeschliffen oder anderweitig vorbehandelt werden müssten.

Die Wahl eines geeigneten Extruders erfolgt anhand der jeweiligen Bedürfnisse. Er kann beispielsweise eine Schnecke in einer runden Bohrung oder zwei achsparallele Schnecken in einer acht-förmigen Bohrung aufweisen (siehe Figur 1a und die Figuren 2 und 3). Doppelschneckenextruder mit kämmenden, gegeneinander laufenden Schnecken arbeiten mit Zwangsförderung. Durch das Ineinandergreifen der Stege in die Gänge der gegenüberliegenden Schnecke wird ein Kammerabschluss erreicht, wodurch das Komposit in C-förmigen Kammern durch die Bohrung transportiert wird. Bei gleichlaufenden Schnecken wird keine absolute Kammerabsperrung erreicht. Das Material kann achtförmig um die beiden Schnecken herum zurückströmen. Dennoch wird durch die Drehbewegung der Schnecken eine ausreichende Vorwärtsbewegung ermöglicht. Die Entgasung ist beim Doppelschneckenextruder mit gegenlaufenden Schnecken besonders günstig. Das Komposit wird von den sich drehenden Schnecken immer wieder von der Entgasungsöffnung fortgerissen und kann somit die Öffnung nicht verstopfen.

Wenn die Schnecken mit gleicher Drehrichtung und Winkelgeschwindigkeit laufen, ergibt sich durch die Geometrie der Schnecken ein gegenseitiges Abstreifen des Materials bei einem genügend engen Spiel zwischen den Schnecken.

Der in Figur 1a) gezeigte Extruder ist aus unterschiedlichen, gleich langen Kompartimenten zusammengesetzt, die jeweils ein eigenes Gehäuse besitzen, das an seine jeweiligen Nachbargehäuse dichtend angekuppelt ist. Die darin laufende(n) Schnecke(n) können aus unterschiedlichen Bereichen wie Knet- oder Förderelementen mit unterschiedlicher Länge und Steigung zusammengesetzt sein. Durch eine geeignete Kombination können sehr homogene Komposite erhalten werden. Günstig ist ein relativ enger "pitch" (Steigung) im Bereich, in dem das Harz eingefüllt wird ("Gehäuse 1"). Dadurch wird die Förderung in Richtung der Düse verbessert. Die Steigung kann im Bereich der Zuführung des Füllstoffs größer gewählt werden ("Gehäuse 2"). Die nachfolgende Strecke im Extruder (hier: "Gehäuse 3", "Gehäuse 4") kann aus reinen Förderelementen (Schnecken) bestehen, oder aber zusätzlich Knetelemente enthalten. Bei der Wahl der Anzahl der Knetelemente kann Zurückhaltung geboten sein, weil die hohe Scherbeanspruchung dazu führen kann, dass das Komposit instabil wird bzw. aushärtet (was man jedoch in manchen Fällen durch die Zugabe z.B. eines Stabilisators oder eines Inhibitors vermeiden kann, der eine zu frühe Aushärtung verhindert). Der Grund hierfür könnte eine zu hohe Scherbeanspruchung durch die Knetelemente sein, die zu Erwärmung und Härtung führen. Knetelemente sollten daher über möglichst nicht mehr als 35%, vorzugsweise über nicht mehr als 25% der Länge der Förderung des Komposits bis zum Verdichtungsbereich vorgesehen sein. Häufig kann auf Knetelemente ganz verzichtet werden. Die Schnecken und Gehäuse sollten gegebenenfalls gegen Abrasion geschützt sein, z.B. wenn abrasive Füllstoffe aus Glas oder Keramik o.ä. eingesetzt werden.

Die Verwendung eines Extruders, insbesondere eines Doppelschneckenextruders hat den Vorteil, dass sowohl das Einarbeiten des Füllstoffs in die Harzmatrix als auch die Formgebung in einem Verfahrensschritt durchgeführt werden können. Dadurch und durch das kontinuierliche Verfahren ergibt sich eine enorme Zeit- und damit Kostenersparnis.

Der aus dem Extruder austretende Komposit-Strang kann in einer ersten Ausgestaltung der Erfindung auf einem Substrat abgelegt werden, beispielsweise einem in der Geschwindigkeit auf die Austrittsgeschwindigkeit angepassten, sich kontinuierlich bewegenden Band oder einer Luftkissenschiene. Ist kein kontinuierlicher Weitertransport vorgesehen, kann der Strang nach Extrusion eines geeigneten Längenstücks (z.B. 0,5 m) abgeteilt und separat abgelegt werden. Alternativ kann das strangförmige Material abgefangen und in Formen gefüllt werden, in denen es die Gestalt des endgehärteten Materials annimmt. In einer hiervon nochmals abweichenden Ausgestaltung kann das strangförmige Material abgefangen und in verschließbare Gefäße abgefüllt werden, in denen es bis zu einer späteren Aushärtung gelagert werden kann. All dies wird weiter unten näher erläutert.

Der gemäß der vorgenannten ersten Ausgestaltung sich bildende Strang ist zur baldigen Aushärtung vorgesehen. Beim Austritt ist er meist noch relativ weich. Es kann sich daher empfehlen, direkt hinter der Düse eine Vorrichtung anzuordnen, mit der das Komposit verfestigt wird. Bei dieser Verfestigung kann es sich entweder um eine Vorhärtung oder aber um die sofortige vollständige Härtung handeln. Wenn das Harz lichtempfindlich ist, kann der Strang beispielsweise mit elektromagnetischer Strahlung im sichtbaren (Blaulicht) oder im UV-Bereich bestrahlt werden. Ist er thermisch härtbar, kann dort eine Wärmekammer angeordnet werden, oder der Strang wird mit IR-Strahlung bestrahlt. Auch Materialien, die redoxinduziert polymerisieren, sollten nach dem Austritt aus dem Extruder thermisch (nach)gehärtet werden. Eine (Vor-)Härtung mit Strahlung ist in **Figur 1****, Teil b**) schematisch gezeigt. Durch diese Vorhärtung erhält der Kompositstrang die benötigte Standfestigkeit/Maßhaltigkeit für eine mögliche oder notwendige Weiterverarbeitung, weshalb sie sehr zweckmäßig ist. Sie ist jedoch kein zwingendes Kriterium: Wird der Strang am Ausgang des Extruders nicht vorgehärtet, lässt er sich beispielsweise substratgestützt in geeignete Formteile geschnitten oder noch als Strang in einen Ofen oder zu einer Belichtungseinrichtung verbringen, wo er thermisch bzw. mit Hilfe elektromagnetischer Strahlung gehärtet wird. Alternativ ist es möglich, den Strang bereits beim Austritt aus der Düse in der gegebenen Strangform endzuhärten oder unmittelbar in Stücke zu zerteilen und sodann, ebenfalls noch in Düsennähe, endzuhärten, was beispielsweise für Komposite geeignet sein kann, die nur über eine lichtinduzierte Polymerisation aushärten und nur einen auf Licht reagierenden Polymerisationsinitiator aufweisen. Ein einziger Härtungsschritt kann alternativ auch thermisch, und dabei ggf. ebenfalls in Düsennähe durchgeführt werden.

Wurde der Strang beim Austritt aus der Düse nur vorgehärtet, erfolgt anschließend eine Endhärtung. Hierfür wird der Strang (oder es werden die bereits geschnittenen Formen) zu der Einrichtung verbracht, in der die Endhärtung erfolgt. Das kann beispielsweise durch den Weitertransport über das Band oder die Luftkissenschiene durch eine nachgeschaltete Härtungseinrichtung geschehen, die eine Härtung durch (Blau-)Licht, UV-Bestrahlung, IR-Bestrahlung oder Wärme bewirkt. Im letzteren Falle kann der Strang bzw. können die Formen durch einen Ofen durchgeführt werden. Wird das Komposit in Form des Strangs gehärtet, wird es im Anschluss daran in die geeignete Form gebracht, im Falle dentaler CAD/CAM-Formen z.B. in kleine Blöcke gesägt. Es besteht aber natürlich auch die Möglichkeit, erst die finale Formgebung vorzunehmen und erst danach eine Endhärtung vorzunehmen. In der Regel werden die Stränge ggf. vor- und in jedem Fall endgehärtet und erst dann mittels Schneideeinheit in Blöcke zerteilt, z.B. gesägt oder mit einem Wasser- oder Laserstrahl geschnitten. Insbesondere für die Herstellung von CAD/CAM-Blöcken für dentale Zwecke ist dies häufig das Mittel der Wahl.

Für eine Reihe von Anwendungen, darunter die Erzeugung von CAD/CAM-Blöcken, kann es sich empfehlen, dass die erhaltenen Stränge recht genaue Geometrien, insbesondere saubere (meist rechtwinklige) Kanten und/oder glatte Oberflächen, haben. Deshalb können die extrudierten Stränge bei Bedarf, d.h. wenn sie die erforderliche Geometrie-Genauigkeit (noch) nicht besitzen, durch eine Einrichtung geführt werden, in der diese Genauigkeit erzielt wird. Hierfür eignet sich beispielsweise eine Schleifeinrichtung.

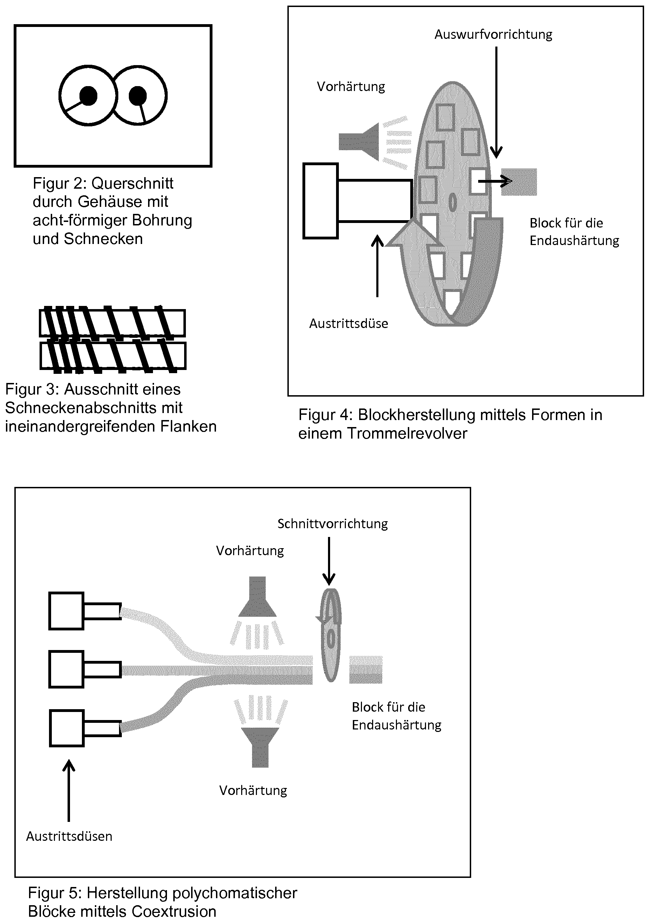

In der Alternative, nach der das strangförmige Material abgefangen und in Formen gefüllt werden soll, in denen es die Gestalt des endgehärteten Materials annimmt, können sich die Formen z.B. in einer Revolvertrommel (siehe **Figur 4**) oder in Stabmagazinen befinden und zeitlich abgestimmt vor der Düse befüllt und ggf. vorgehärtet oder aber gleich in den Formen endgehärtet werden. Findet keine Vor- oder Endhärtung statt, werden die Formen für eine anschließende Härtung sodann von der Düse entfernt. Eine Möglichkeit der Endhärtung in den Formen besteht in der Ofenhärtung. Die Trommeln bzw. Stabmagazine werden dafür in einen entsprechenden Ofen gelegt. Durch den Druck an der Düse ist es möglich, auch hochgefüllte Komposite in Formen zu füllen.

Mit den vorstehend beschriebenen Ausführungsformen lassen sich monolithische Körper herstellen. Im Falle dentaler Blöcke, die z.B. später in einem CAD/CAM-Verfahren endbearbeitet werden sollen, handelt es sich dabei um einfarbige, sogenannte monochromatische Blöcke. Für eine hochwertigere Ästhetik können auch mehrfarbige, sogenannte polychromatische Blöcke erzeugt werden, beispielsweise über Coextrusion. Ein Beispiel hierfür ist in **Figur 5** schematisch dargestellt. Hierfür werden Komposite mit unterschiedlicher Farbe bzw. Transluzenz in jeweils einem Extruder hergestellt und über eine Schlitzdüse, die den Maßen der jeweiligen Schicht entspricht, gefördert. Die Düsen der einzelnen Komposite sind so angelegt, dass die einzelnen Schichten übereinander abgelegt/gestapelt werden. Die Vor- und Endhärtung erfolgt analog zu der Herstellung der monochromatischen Blöcke. Eine weitere Möglichkeit besteht in der Herstellung/Extrusion und Vorhärtung der ersten Schicht. Diese wird dann erneut unter der Extruderdüse entlanggeführt und die zweite Schicht aus einem anderen Material wird darauf abgelegt und vorgehärtet.

Dieser Vorgang wird entsprechend der Schichtzahl wiederholt. Anstelle polychromatischer Blöcke können natürlich auch Blöcke mit anderen über ihre Gesamtdicke hinweg variierenden physikalischen Eigenschaften erzeugt werden, beispielsweise mit unterschiedlichen E-Modulen. Die Erfindung ist auf die Geometrie der gezeigten Coextrusion nicht beschränkt. Andere Geometrien sind möglich, beispielsweise die Coextrusion durch ringförmig ineinander liegende Düsen.

Voranstehend wurde die Erfindung allgemein und unter Bezugnahme auf die Dentaltechnik erläutert. Der Vorteil zur klassischen Herstellung und Abfüllung von Dentalkompositen besteht im Verfahren selbst. Da es sich bei der Extrusion um ein kontinuierliches Verfahren handelt, fallen viele nicht-kontinuierliche Arbeitsschritte in der Kompositherstellung wie z.B. die Füllstoffeinarbeitung/Homogenisierung mittels Planetenmischer, Speedmixer etc. und das manuelle/halbautomatische Befüllen der Applikationsspritzen etc. weg.

Die Vorteile der vorliegenden Erfindung liegen in der Bereitstellung eines einfachen und kontinuierlich durchführbaren Verfahrens zur Komposit- bzw. Blockherstellung, insbesondere für dentale Anwendungen. So können ausgehärtete CAD/CAM-Blöcke (monochrom und polychrom) erzeugt werden. Das Verfahren ist einer Vollautomatisierung mit hohem Produktionsdurchsatz zugänglich, wobei eine gleichbleibende Qualität effektiv sichergestellt werden kann. Die Erfindung stellt damit im Dentalbereich einen Ersatz des bisher arbeits-/zeitaufwändigen und somit kostenintensiven Verfahrens aufgrund eines effektiver gestalteten Prozesses zur Verfügung. Man erhält chemisch/physikalisch belastungsstabile biokompatible Materialsysteme ohne relevante Blasen/Lufteinschlüsse etc.

Da das Verfahren für den Einsatz von Kompositen auf Basis von polymerisierbaren Matrixsystemen (z.B. organische Monomere/Oligomere, ORMOCER®e, etc.) ausgelegt ist, lassen sich die nachstehenden besonderen Vorteile realisieren:
- Eine dem natürlichen Zahn angepasste Ästhetik/Transluzenz durch Mehrschichtaufbau
- Die Möglichkeit des Einsatzes unterschiedlicher Matrixsystemtypen/Komposittypen für verschiedene Schichten
- eine hohe Abrasionsresistenz bei guter chemischer/physikalischer Stabilität sowie guter Reparaturfähigkeit.

Werden anorganisch-organische Hybridmaterialien wie Kieselsäure(hetero)polykondensate (als Marke "ORMOCER®e" geschützt für die Fraunhofer Gesellschaft) eingesetzt, können diese mit oder ohne Zusatz weiterer copolymerisierender organischer Monomere eingesetzt werden. Monomerfreie Systeme stellen eine besonders biokompatible Werkstoffbasis dar, wodurch eine toxikologische und allergologische Unbedenklichkeit für den Einsatz als Zahnersatzmaterial gegeben ist.

Mit dem vorliegend beschriebenen neuartigen Verfahren können in Verbindung mit einem belastungsstabilen Werkstoffsystem CAD/CAM-Blöcke für Ein-/ Mehrschichtkronen, Inlays, Onlays und Veneers (Verblendschalen) etc. in einem kontinuierlichen Verfahren angefertigt werden. Weiterhin können z.B. die momentan in vielen Arbeitsschritten hergestellten Füllungskomposite automatisiert/kontinuierlich hergestellt und abgefüllt werden.

### Ausführungsbeispiele

Zur Herstellung von CAD/CAM-Blöcken mittels Extrusion wurde ein Extruder mit insgesamt 6 Gehäusen wie in Fig. 1a) gezeigt verwendet. Der Extruder wurde mit einer Geschwindigkeit von 250 rpm (Kompositzusammensetzung 1) bzw. 60-100 rpm (Kompositzusammensetzungen 2 bis 5) gefahren. Anschließend wurde über eine Schlauchpumpe die Flüssigdosierung (0,3 kg/h) gestartet. Die Harzzugabe erfolgte dabei drucklos in Gehäuse 1 über ein offenes Röhrchen mit einem Bohrungsdurchmesser von 2mm. Nach ca. 5 min erfolgte dann auch die Feststoffdosierung mit dem Füllstoff. Hierfür wurde in Gehäuse 2 das Dentalglas von oben über einen Einlauftrichter zudosiert. In den geschlossenen Gehäusen 3 und 4 erfolgte das Mischen beider Komponenten zu einer homogenen Masse, die in Gehäuse 5 mittels Vakuum (ca. -600 mbar) entgast wurde, wobei Gehäuse 6 zum Druckaufbau diente. Um sicher zu stellen, dass die Temperatur des Komposites im Extruder nicht auf ≤ 65°C anstieg, wurden die Gehäuse 2 bis 6 auf 30°C temperiert. Nach dem letzten Gehäuse folgte ein Übergangsstück zur Formgebung des Stranges (rechteckig, 12 x 14 mm). Aufgrund der langsamen Vorschubbewegung wurde der Strang vorerst manuell abgenommen. Der Strang wurde bei Austritt aus dem Röhrchen mit drei Blaulichtlampen bestrahlt und manuell auf einer sauberen Ablagefläche abgelegt. Abschließend fand eine thermische Nachhärtung der Stränge im Ofen für 4 h bei 100°C statt.

### Kompositzusammensetzungen:

### Zusammensetzung 1

28 Gew.-% Harzsystem aus:
   40 Gew.-% Bis-GMA (Bisphenol-A-Dimethacrylat),
   40 Gew.-% UDMA (Urethandimethacrylat) und
   20 Gew.-% TEGDMA (Triethylenglycoldimethacrylat),
   das mit 1 Gew.-% Lucirin TPO (LTPO, 2,4,6-Trimethylbenzoyldiphenylphosphinoxid, lichtempfindlicher Initiator) und 2 Gew.-% DBPO (Dibenzoylperoxid, thermischer Initiator) versetzt wurde, mit einer Viskosität von ca. 2,7 Pas und
72 Gew.-% Füllstoff, silanisiert (Primärpartikelgröße D₅₀= 1 µm)

### Zusammensetzung 2

25 Gew.-% Harzsystem aus:
   40 Gew.-% Bis-GMA (Bisphenol-A-Dimethacrylat),
   40 Gew.-% UDMA (Urethandimethacrylat) und
   20 Gew.-% TEGDMA (Triethylenglycoldimethacrylat),
   das mit 1 Gew.-% Lucirin TPO (LTPO, 2,4,6-Trimethylbenzoyldiphenylphosphinoxid, lichtempfindlicher Initiator) und 2 Gew.-% DBPO (Dibenzoylperoxid, thermischer Initiator) versetzt wurde, mit einer Viskosität von ca. 2,7 Pas und
75 Gew.-% Füllstoffanteil, silanisiert (Schott) bestehend aus
   - 67 Gew.-% Ultrafine, Primärpartikelgröße: 0,7 µm
   - 33 Gew.-% Standard Grind K6, Primärpartikelgröße: 3,0 µm

### Zusammensetzung 3

28 Gew.-% Harzsystem aus:
   40 Gew.-% Bis-GMA (Bisphenol-A-Dimethacrylat),
   40 Gew.-% UDMA (Urethandimethacrylat) und
   20 Gew.-% TEGDMA (Triethylenglycoldimethacrylat),
   das mit 1 Gew.-% Lucirin TPO (LTPO, 2,4,6-Trimethylbenzoyldiphenylphosphinoxid, lichtempfindlicher Initiator) und 2 Gew.-% DBPO (Dibenzoylperoxid, thermischer Initiator) versetzt wurde, mit einer Viskosität von ca. 2,7 Pas und
72 Gew.-% Füllstoffanteil, silanisiert (Quarzwerke) bestehend aus
   - 50 Gew.-% Microspar 1351, Primärpartikelgröße: 0,8 µm
   - 50 Gew.-% Microspar 1351, Primärpartikelgröße: 2,0 µm

### Zusammensetzung 4

25 Gew.-% ORMOCER®-Harzsystem A
   das mit 1 Gew.-% Lucirin TPO (LTPO, 2,4,6-Trimethylbenzoyldiphenylphosphinoxid, lichtempfindlicher Initiator) und 2 Gew.-% DBPO (Dibenzoylperoxid, thermischer Initiator) versetzt wurde, mit einer Viskosität von ca. 4,0 Pas und
75 Gew.-% Füllstoffanteil, silanisiert (Schott) bestehend aus
   - 67 Gew.-% Ultrafine, Primärpartikelgröße: 0,7 µm
   - 33 Gew.-% Standard Grind K6, Primärpartikelgröße: 3,0 µm

### Zusammensetzung 5

28 Gew.-% ORMOCER®-Harzsystem B
   das mit 1 Gew.-% Lucirin TPO (LTPO, 2,4,6-Trimethylbenzoyldiphenylphosphinoxid, lichtempfindlicher Initiator) und 2 Gew.-% DBPO (Dibenzoylperoxid, thermischer Initiator) versetzt wurde, mit einer Viskosität von ca. 25,0 Pas und
72 Gew.-% Füllstoffanteil, silanisiert (Schott) bestehend aus
   - 67 Gew.-% Ultrafine, Primärpartikelgröße: 0,7 µm
   - 33 Gew.-% Standard Grind K6, Primärpartikelgröße: 3,0 µm

### Harzsystem A (Synthese gemäß DE 44 16 857 C1)

Zur Vorlage von 125,0 g (0,503 mol) 3-Glycidyloxypropylmethyldiethoxysilan werden unter trockener Atmosphäre Triphenylphosphin als Katalysator, BHT (3,5-Di-tert.-butyl-4-hydroxytoluol) - als Stabilisator und anschließend 47,35 g (0,550 mol) Methacrylsäure zugetropft und bei 80°C gerührt (ca. 24 h). Die Umsetzung kann über die Abnahme der Carbonsäurekonzentration mittels Säuretitration sowie dem Epoxidumsatz mittels Epoxidtitration/NMR verfolgt werden. Nach Zugabe von Essigester (1000 ml/mol Silan) und H₂O zur Hydrolyse mit HCl als Kat. wird bei 30°C gerührt. Die Aufarbeitung erfolgt nach mehrtägigem Rühren durch Ausschütteln mit wässriger NaOH und Wasser und Filtration über hydrophobierten Filter. Es wird zunächst abrotiert und anschließend mit Ölpumpenvakuum abgezogen. Es resultiert ein flüssiges Harz mit einer Viskosität von ≈ 3 - 5 Pa·s bei 25°C sowie einer Brechzahl n_{D} ≈ 1,477.

### Harzsystem B (Synthese gemäß DE 103 49766 A1)

### Grundreaktionsprinzip:

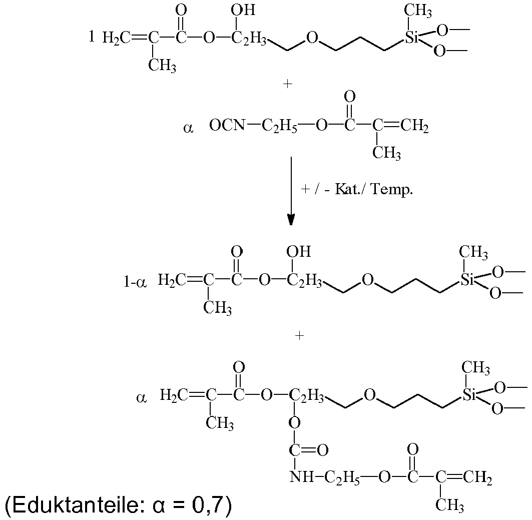

Zur Vorlage von 130,3 g (0,50 mol) Harzsystem A und 0,09 g BHT werden unter trockener Atmosphäre bei 30°C unter Rühren 54,3 g Methacrylsäureisocyanatoethylester (0,70 mol) zugetropft und bei 30°C weitergerührt. Nach vollständiger Umsetzung resultiert ein flüssiges Harz mit einer Viskosität von ca. 22 - 28 Pa·s bei 25°C.

## Patentansprüche

1. Verfahren zum Mischen und Formen von Kompositen aus einer Reaktivharzmatrix, die beim Aushärten duromere Eigenschaften annimmt, und einem Füllstoff, **gekennzeichnet durch** die folgenden Schritte:
(a) Dosieren eines flüssigen oder pastösen Reaktivharzes in einen Extruder,
(b) Zugabe von Füllstoff zu dem im Extruder befindlichen Reaktivharz, wobei das Mischungsverhältnis von Harz zu Füllstoff so gewählt wird, dass die Komposite einen Füllstoffgehalt von 45 bis 90 Gew.-% aufweisen,
(c) Vermischen der Komponenten mit Hilfe von einer oder mehreren Extruderschnecken unter Ausbildung eines Komposits,
(d) Fördern des Komposits in einen Bereich des Extruders, in dem es aufgestaut wird,
(e) Entgasen des aufgestauten Komposits, und
(f) Fördern des entgasten Komposits durch eine geeignet geformte Düse aus dem Extruder heraus,
(g) Härten des aus dem Extruder getretenen Komposits durch Belichten und/oder Erwärmen, und
(h) Zerteilen des Strangs in die gewünschte Form,
wobei die Schritte (g) und (h) in beliebiger Reihenfolge durchgeführt werden können.

2. Verfahren nach Anspruch 1, worin der Härtungsschritt (g) aus einer Vorhärtung und einer finalen Aushärtung besteht.

3. Verfahren nach Anspruch 1 oder 2, worin die Vorhärtung unmittelbar hinter der Extruderdüse durch Belichten mit sichtbarem oder UV-Licht oder Behandeln mit IR-Strahlung erfolgt.

4. Verfahren nach einem der voranstehenden Ansprüche, **dadurch gekennzeichnet, dass** der Extruder außenseitig auf einer Temperatur gehalten wird, die mindestens etwa 10K, vorzugsweise mindestens 30K unter der Temperatur liegt, bei der das Reaktivharz zu polymerisieren beginnt.

5. Verfahren nach einem der voranstehenden Ansprüche, worin in Schritt (a) das Reaktivharz druckfrei und/oder scherungsfrei zudosiert wird.

6. Verfahren nach einem der voranstehenden Ansprüche, worin in Schritt (e) der Druck um nicht mehr als 800 mbar, vorzugsweise nicht mehr als 650 mbar (hPa) unter den Normaldruck abgesenkt wird.

7. Verfahren nach einem der voranstehenden Ansprüche, worin die Reaktivharzmatrix organisch polymerisierbare Gruppen enthält, die ausgewählt sind unter nichtaromatischen C=C-Doppelbindungen und gespannten Ringsystemen.

8. Verfahren nach einem der voranstehenden Ansprüche, worin die Reaktivharzmatrix einen oder mehrere Polymerisationsinitiatoren enthält, die unter UV-Strahlung und/oder unter Strahlung im sichtbaren Bereich und/oder unter thermischer Anregung eine Polymerisationsreaktion initiieren.

9. Verfahren nach einem der voranstehenden Ansprüche, worin die Reaktivharzmatrix ein organisch modifiziertes Kieselsäure(hetero)polykondensat aufweist oder daraus besteht.

10. Verfahren nach einem der voranstehenden Ansprüche, worin der Füllstoff ausgewählt ist aus einem ggf. oberflächensilanisierten Dentalglas oder einer Mischung mehrerer solcher Gläser.

11. Verfahren nach einem der Ansprüche 1 bis 10, worin das geformte und gehärtete Komposit ein für die CAD/CAM-Bearbeitung geeigneter Formkörper ist.

12. Verfahren nach Anspruch 11, worin der für die CAD/CAM-Bearbeitung geeignete Formkörper für die Herstellung von Kronen, Brücken, Inlays, Onlays oder Veneers vorgesehen ist.

## Claims

1. A process for mixing and molding composites of a reactive resin matrix, which assumes duromeric properties upon curing, and a filler, **characterized by** the following steps:
(a) dosing a liquid or pasty reactive resin into an extruder,
(b) adding filler to the reactive resin present in the extruder, wherein the mixing ratio of resin to filler is selected such that the composites have a filler content of 45 to 90 % by weight,
(c) mixing the components with the aid of one or more extruder screws to form a composite,
(d) conveying the composite into a region of the extruder in which it is dammed,
e) degassing the dammed composite, and
(f) conveying the degassed composite out of the extruder through a suitably shaped die,
(g) curing the composite exited from the extruder by exposure and/or heating, and
(h) dividing the strand into the desired shape,
wherein the steps (g) and (h) can be carried out in any order.

2. The process according to claim 1, wherein the curing step (g) consists of a pre-curing and a final curing.

3. The process according to claim 1 or 2, wherein the pre-curing takes place directly after the extruder die by exposure to visible or UV light or treatment with IR radiation.

4. The process according to any one of the preceding claims, **characterized in that** the extruder is kept externally at a temperature which is at least about 10K, preferably at least 30K below the temperature at which the reactive resin begins to polymerize.

5. The process according to any one of the preceding claims, wherein in step (a) the reactive resin is added pressure-free and/or shear-free.

6. The process according to any one of the preceding claims, wherein in step (e) the pressure is lowered below normal pressure by not more than 800 mbar, preferably not more than 650 mbar (hPa).

7. The process according to any one of the preceding claims, wherein the reactive resin matrix contains organically polymerizable groups selected from non-aromatic C=C double bonds and tense ring systems.

8. The process according to any one of the preceding claims, wherein the reactive resin matrix contains one or more polymerization initiators which initiate a polymerization reaction under UV radiation and/or under radiation in the visible range and/or under thermal excitation.

9. The process according to any one of the preceding claims, wherein the reactive resin matrix comprises or consists of an organically modified silica (hetero)polyconden sate.

10. The process according to any one of the preceding claims, wherein the filler is selected from an optionally surface-silanized dental glass or a mixture of several such glasses.

11. The process according to any of Claims 1 to 10, wherein the molded and cured composite is a molded body suitable for CAD/CAM machining.

12. The process according to claim 11, wherein the molded body suitable for CAD/CAM processing is intended for the production of crowns, bridges, inlays, onlays or veneers.

## Revendications

1. Procédé de mélange et de mise en forme de composites issus d'une matrice de résine réactive, qui acquiert en durcissant des propriétés d'un duromère, et d'une charge, **caractérisé par** les étapes suivantes:
(a) doser une résine réactive liquide ou pâteuse dans une extrudeuse;
(b) ajouter une charge à la résine réactive se trouvant dans l'extrudeuse, le rapport de mélange de la résine à la charge étant choisi de telle manière que les composites présentent une teneur en charge de 45 à 90 % en poids,
(c) mélanger les composants à l'aide d'une ou de plusieurs vis sans fin d'extrudeuse avec formation d'un composite,
(d) transporter le composite dans une zone de l'extrudeuse dans laquelle il est accumulé,
(e) dégazer le composite accumulé, et
(f) transporter le composite dégazé hors de l'extrudeuse au moyen d'une buse de forme appropriée,
(h) durcir le composite extrait hors de l'extrudeuse par illumination et/ou chauffage, et
(h) diviser le cordon à la forme désirée,
dans lequel les étapes (g) et (h) peuvent être exécutées dans un ordre arbitraire.

2. Procédé selon la revendication 1, dans lequel l'étape de durcissement (g) se compose d'un pré-durcissement et d'un durcissement final.

3. Procédé selon une revendication 1 ou 2, dans lequel on effectue le pré-durcissement immédiatement après la buse d'extrudeuse par illumination avec une lumière visible ou UV ou par traitement avec un rayonnement IR.

4. Procédé selon l'une quelconque des revendications précédentes, **caractérisé en ce que** l'extrudeuse est maintenue extérieurement à une température, qui est située au moins environ 10 K, de préférence au moins 30 K en dessous de la température, à laquelle la résine réactive commence à se polymériser.

5. Procédé selon l'une quelconque des revendications précédentes, dans lequel on dose à l'étape (a) la résine réactive sans pression et/ou sans cisaillement.

6. Procédé selon l'une quelconque des revendications précédentes, dans lequel à l'étape (e) on abaisse la pression de pas plus de 800 mbar, de préférence de pas plus de 650 mbar (hPa), en dessous de la pression normale.

7. Procédé selon l'une quelconque des revendications précédentes, dans lequel la matrice de résine réactive contient des groupes organiquement polymérisables, qui sont choisis parmi les doubles liaisons C=C non aromatiques et les systèmes d'anneau tendus.

8. Procédé selon l'une quelconque des revendications précédentes, dans lequel la matrice de résine réactive contient un ou plusieurs initiateur(s) de polymérisation, qui déclenchent une réaction de polymérisation sous un rayonnement UV et/ou sous un rayonnement dans le domaine visible et/ou par excitation thermique.

9. Procédé selon l'une quelconque des revendications précédentes, dans lequel la matrice de résine réactive présente un (hétéro)polycondensat d'acide silicique organiquement modifié ou en est constituée.

10. Procédé selon l'une quelconque des revendications précédentes, dans lequel la charge est choisie parmi un verre dentaire éventuellement silanisé en surface ou un mélange de plusieurs de tels verres.

11. Procédé selon l'une quelconque des revendications 1 à 10, dans lequel le composite mis en forme et durci est un corps moulé convenant pour l'usinage CAD/CAM.

12. Procédé selon la revendication 11, dans lequel le corps moulé convenant pour l'usinage CAD/CAM est prévu pour la fabrication de couronnes, de ponts, d'inlays, d'onlays ou de facettes dentaires.
